# EUROPÄISCHE PATENTSCHRIFT

(11) **EP 0 662 814 B1**
(45) Veröffentlichungstag und Bekanntmachung des Hinweises auf die Patenterteilung: **17.07.1996**
(21) Anmeldenummer: 93914723.7
(22) Anmeldetag: 24.06.1993
(51) Int. Cl.: A61K 7/06

(54) **HAARBEHANDLUNGSMITTEL**
HAIR TREATMENT AGENT
PRODUIT DE TRAITEMENT DES CHEVEUX

(30) Priorität: 29.09.1992 DE 4232506; 29.09.1992 DE 4232512
(43) Veröffentlichungstag der Anmeldung: 19.07.1995
(73) Patentinhaber: Henkel Kommanditgesellschaft auf Aktien, 40191 Düsseldorf (DE)
(72) Erfinder: SEIDEL, Kurt, D-4000 Düsseldorf 13 (DE); MÜLLER, Reinhard, D-5140 Erkelenz 7 (DE); HOLLENBERG, Detlef, D-4006 Erkrath (DE); EHLERT, Manuela, D-5090 Leverkusen 3 (DE)
(86) Internationale Anmeldenummer: EP9301624
(87) Internationale Veröffentlichungsnummer: WO9407455

(56) Entgegenhaltungen:
- EP-A- 0 342 056
- EP-A- 0 485 212
- DE-A- 3 330 628

## Beschreibung

Die Erfindung betrifft Zubereitungen zur Haarbehandlung mit einer speziellen Wirkstoffkombination.

Die Reinigung und Pflege der Haare ist ein wichtiger Bestandteil der menschlichen Körperpflege. Sowohl die Reinigung der Haare mit Shampoos als auch die dekorative Gestaltung der Frisur beispielsweise durch Färben oder Dauerwellen sind Eingriffe, die die natürliche Struktur und die Eigenschaften der Haare beeinflussen. So können anschließend an eine solche Behandlung beispielsweise die Naß- und Trockenkämmbarkeit des Haares, der Halt und die Fülle des Haares unbefriedigend sein oder die Haare einen erhöhten Spliß aufweisen. Weiterhin ist die gleichmäßige Verteilung der mit Haarfärbemitteln aufgebrachten Farbstoffe insbesondere auf stark vorgeschädigtem Haar häufig sehr problematisch.

Es ist daher seit langem üblich, die Haare einer speziellen Nachbehandlung Zu unterziehen. Dabei werden, üblicherweise in Form einer Spülung, die Haare mit speziellen Wirkstoffen, beispielsweise quaternären Ammoniumsalzen oder speziellen Polymeren, behandelt. Durch diese Behandlung werden je nach Formulierung Kämmbarkeit, Halt und Fülle der Haare verbessert und die Splißrate verringert. Dennoch sind die Ergebnisse für colorierte, vorgeschädigte Haare noch nicht befriedigend.

Es wurde nun überraschenderweise gefunden, daß Mittel mit einer Kombination aus Vertretern zweier bestimmter, jeweils für ihre Anwendbarkeit in Haarbehandlungsmitteln bekannter Wirkstoffklassen die Eigenschaften von stark vorgeschädigtem, coloriertem Haar unerwartet gut beeinflussen. Bei Behandlung von stark vorgeschädigten, colorierten Haaren mit diesen Mitteln tritt vor allem ein starker Egalisierungseffekt unter überraschend hoher Farbschonung auf. Damit wird es möglich, diese Haare weitaus häufiger zu Reinigen und Nachzubehandeln, bevor eine erneute Einfärbung notwendig wird. Außerdem erlaubt die gute biologische Abbaubarkeit der Wirkstoffkombination die Formulierung von Produkten mit aus ökologischen Gesichtspunkten geforderter guter Abbaubarkeit.

Gegenstand der Erfindung ist daher die Verwendung einer wäßrigen Zubereitung enthaltend eine Wirkstoffkombination, bestehend aus
(A) alkoxylierten Wollwachsalkoholen und
(B) einer Pyrrolidoncarbonsäure und/oder einem Pyrrolidoncarbonsäurederivat der allgemeinen Formel (I), 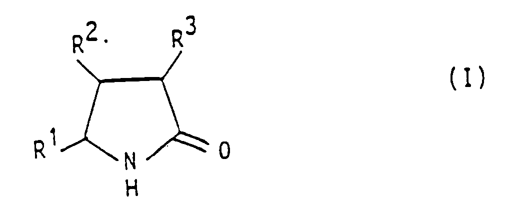 in der mindestens einer der Substituenten R¹ bis R³ steht für eine Gruppe -COOR⁴, in der R⁴ Wasserstoff, ein Alkalimetallion, ein Erdalkalimetallion oder ein Ammoniumion ⁺NHR⁵R⁶R⁷ ist, in dem R⁵ bis R⁷ unabhängig voneinander Wasserstoff, Alkylgruppen mit 1 bis 22 C-Atomen, Hydroxyalkylgruppen mit 1 bis 4 C-Atomen, Alkenylgruppen mit 2 bis 22 C-Atomen, Acylgruppen mit 2 bis 22 C-Atomen oder gegebenenfalls substituierte aromatische Gruppen mit 6 bis 10 C-Atomen sind, und die restlichen Substituenten R¹ bis R³ für Wasserstoff oder Alkylgruppen mit 1 bis 4 C-Atomen stehen,

sowie üblichen kosmetischen Bestandteilen zur Behandlung, insbesondere zur Reinigung und Pflege, von Haaren.

Sowohl die Verwendung von ethoxylierten Wollwachsalkoholen (s. z.B. H. P. Fiedler, Lexikon der Hilfsstoffe für Pharmazie, Kosmetik und angrenzende Gebiete, 2. Auflage, Editio Cantor Aulendorf, 1981, Seite 724) als auch von Verbindungen der Formel (I) (s. z.B. deutsche Offenlegungsschrift DE-A- 41 31 898.6) zur Haarbehandlung ist bekannt.

Aus der deutschen Patentschrift DE-C2-33 30 628 (insb. Beispiel 2) sind Hautschutz- und -pflegelotionen bekannt, die ethoxylierte Wollfettalkohole und das Natriumsalz der Pyrrolidoncarbonsäure enthalten. Dieser Druckschrift ist aber keinerlei Hinweis auf Wirkungen dieser Kombination am Haar zu entnehmen.

Wollwachsalkohole, die auch als Lanolinalkohole bezeichnet werden, sind komplexe Mischungen aus Sterinen und aliphatischen Alkoholen, die aus dem Wollwachs gewonnen werden. Hauptbestandteil der Wollwachsalkohole sind Cholesterin, Lanosterin und von diesen abgeleitete Sterine, C₁₈-C₃₀-n-Alkohole, C₁₆-C₂₆-iso-Alkohole, Alkandiole und iso-Alkandiole.

Durch Umsetzung dieser Mischungen mit Alkylenoxiden, insbesondere Ethylenoxid und Propylenoxid, werden die alkoxylierten Verbindungen erhalten, die u.a. als Emulgatoren, Lösungsvermittler, Feuchthaltemittel und Konditioniermittel bekannt sind. Diese Produkte stellen üblicherweise keine einheitlich alkoxylierten Verbindungen dar, sondern weisen in Abhängigkeit von dem gewählten Alkoxylierungsverfahren eine entsprechende Homologenverteilung auf. Der angegebene Alkoxylierungsgrad bezieht sich dabei jeweils auf die molaren Ausgangsmengen an Wollwachsalkohol und Alkylenoxid.

Ethoxylierte Wollwachsalkohole sind im Rahmen der Erfindung bevorzugt. Besonders bevorzugt sind dabei Ethoxylierungsgrade von 1 bis 50, insbesondere von 2 bis 30.

Die Verbindungen (B) sind Derivate des 2-Pyrrolidons. Bevorzugte Derivate sind die 2-Pyrrolidon-3-, -4- und -5-carbonsäure und deren Salze. Bevorzugte Salze dieser Verbindungen sind die Natrium-, Kalium-, Calcium-, Magnesium- und solche Ammoniumsalze, bei denen das Ammoniumion neben Wasserstoff eine bis drei C₁- bis C₄-Alkylgruppen trägt.

Besonders bevorzugte Verbindungen (A) sind die 2-Pyrrolidon-5-carbonsäure und deren Salze. Das Natriumsalz ist ganz besonders bevorzugt.

Die erfindungsgemäß verwendeten Zubereitungen enthalten die alkoxylierten Wollwachsalkohole (A) bevorzugt in Mengen von 0,1 bis 5 Gew.-%, bezogen auf die gesamte Zubereitung. Die Verbindungen (B) sind ebenfalls bevorzugt in Mengen von 0,1 bis 5 Gew.-%, enthalten.

Für die Haarbehandlung besonders gut geeignet sind solche Zubereitungen, die neben den obligatorischen Komponenten (A) und (B) zusätzlich noch eine polymere Verbindung enthalten. Bevorzugt sind Polymere aus der Gruppe der kationischen, anionischen und amphoteren Polymeren.

Geeignete kationische Polare enthalten üblicherweise ein quartäres Stickstoffatom, beispielsweise in Form einer Ammoniumgruppe. Bevorzugte kationische Polymere sind beispielsweise
- quaternisierte Cellulose-Derivate, wie sie unter den Bezeichnungen Celquat^{R} und Polymer JR^{R} im Handel erhältlich sind. Die Verbindungen Celquat H 100, Celquat L 200 und Polymer JR^{R}400 sind bevorzugte quaternierte Cellulose-Derivate.
- Polysiloxane mit quaternären Gruppen,
- Polymere Dimethyldiallylammoniumsalze und deren Copolymere mit Estern und Amiden von Acrylsäure und Methacrylsäure. Die unter den Bezeichnungen Merquat^{R}100 (Poly(dimethyldiallylammoniumchlorid)) und Merquat^{R}550 (Dimethyldiallylammoniumchlorid-Acrylamid-Copolymer) im Handel erhältlichen Produkte sind Beispiele für solche kationischen Polymere.
- Copolymere des Vinylpyrrolidons mit quaternierten Derivaten des Dialkylaminoacrylats- und methacrylats, wie beispielsweise mit Diethylsulfat quaternierte Vinylpyrrolidon-Dimethylaminomethacrylat-Copolymere. Solche Verbindungen sind unter den Bezeichnungen Gafquat^{R}734 und Gafquat^{R}755 im Handel erhältlich.
- Vinylpyrrolidon-Methoimidazoliniumchlorid-Copolymere, wie sie unter der Bezeichnung Luviquat^{R} angeboten werden.
- quaternierter Polyvinylalkohol
   sowie die unter den Bezeichnungen
- Polyquaternium 2,
- Polyquaternium 17,
- Polyquaternium 18 und
- Polyquaternium 27 bekannten Polymeren mit quartären Stickstoffatomen in der Polymerhauptkette.

Beispiele für erfindungsgemäß geeignete anionische Polymere sind:
- Polyacryl- und Polymethacrylsäuren, deren Salze, deren Copolymere mit Acrylsäure- und Methacrylsäureestern und -amiden und deren Derivate, die durch Kreuzvernetzung mit polyfunktionellen Agentien erhalten werden. Verbindungen dieser Art sind beispielsweise unter den Bezeichnungen Carbopol^{R}934, Carbopol^{R}934P, Carbopol^{R}940, Carbopol^{R}950 Carbopol^{R}980 und Hostacerin^{R}PN 73 erhältlich.
- Polyoxycarbonsäuren, wie Polyketo- und Polyaldehydocarbonsäuren und deren Salze, wie beispielsweise POC^{R}HS 5060 und POC^{R}AS 5060.
- Polymere und Copolymere der Crotonsäure mit Estern und Amiden der Acryl- und der Methacrylsäure, wie Vinylacetat-Crotonsäure- und Vinylacetat-Vinylpropionat-Crotonsäure-Copolymere. Verbindungen dieser Art sind als Luviset^{R}CA-66 und Luviset^{R}CAP erhältlich.

Unter dem Oberbegriff Ampho-Polymere sind amphotere Polymere, d.h. Polymere, die im Molekül sowohl freie Aminogruppen als auch freie -COOH- oder SO₃H-Gruppen enthalten und zur Ausbildung innerer Salze befähigt sind, zwitterionische Polymere, die im Molekül quartäre Ammoniumgruppen und - COO⁻- oder -SO₃⁻-Gruppen enthalten, und solche Polymeren zusammengefaßt, die -COOH- oder SO₃H-Gruppen und quartäre Ammoniumgruppen enthalten.

Ein Beispiel für ein erfindungsgemäß einsetzbares Amphopolymer ist das unter der Bezeichnung Amphomer^{R} erhältliche Acrylharz, das ein Copolymeres aus tert.-Butylaminoethylmethacrylat, N-(1,1,3,3-Tetramethylbutyl)acrylamid sowie zwei oder mehr Monomeren aus der Gruppe Acrylsäure, Methacrylsäure und deren einfachen Estern darstellt.

Weitere erfindungsgemäß einsetzbare Ampho-Polymere sind die in der britischen Offenlegungsschrift 2 104 091, der europäischen Offenlegungsschrift 47 714, der europäischen Offenlegungsschrift 217 274, der europäischen Offenlegungsschrift 283 817 und der deutschen Offenlegungsschrift 28 17 369 genannten Verbindungen.

Bevorzugt eingesetzte Ampho-Polymere ist solche Polymerisate, die sich im wesentlichen zusammensetzen aus
(a) Monomeren mit quartären Ammoniumgruppen der allgemeinen Formel (II),

   R⁸-CH=CR⁹-CO-Z-(CₙH₂ₙ)-N⁽⁺⁾R¹⁰R¹¹R¹² A⁽⁻⁾ (II)

   in der R⁸ und R⁹ unabhängig voneinander stehen für Wasserstoff oder eine Methylgruppe und R¹⁰, R¹¹ und R¹² unabhängig voneinander für Alkylgruppen mit 1 bis 4 Kohlenstoff-Atomen, Z eine NH-Gruppe oder ein Sauerstoffatom, n eine ganze Zahl von 2 bis 5 und A⁽⁻⁾ das Anion einer organischen oder anorganischen Säure ist
   und
(b) monomeren Carbonsäuren der allgemeinen Formel (III),

   R¹³-CH=CR¹⁴-COOH (III)

   in denen R¹³ und R¹⁴ unabhängig voneinander Wasserstoff oder Methylgruppen sind.

Diese Verbindungen können sowohl direkt als auch in Salzform, die durch Neutralisation der Polymerisate, beispielsweise mit einem Alkalihydroxid, erhalten wird, erfindungsgemäß eingesetzt werden. Bezüglich der Einzelheiten der Herstellung dieser Polymerisate wird ausdrücklich auf den Inhalt der deutschen Offenlegungsschrift 39 29 973 Bezug genommen. Ganz besonders bevorzugt sind solche Polymerisate, bei denen Monomere des Typs (a) eingesetzt werden, bei denen R¹⁰, R¹¹ und R¹² Methylgruppen sind, Z eine NH-Gruppe und A⁽⁻⁾ ein Halogenid-, Methoxysulfat- oder Ethoxysulfat-Ion ist; Acrylamidopropyl-trimethyl-ammoniumchlorid ist ein besonders bevorzugtes Monomeres (a). Als Monomeres (b) für die genannten Polymerisate wird bevorzugt Acrylsäure verwendet.

Bevorzugt enthalten die erfindungsgemäßen Zubereitungen die kationischen, anionischen oder amphoteren Polymere in Mengen von 0,1 bis 5 Gew.-%, bezogen auf die gesamte Zubereitung.

Die Verwendung von Zubereitungen, die ein Polymer aus der Gruppe der amphoteren Polymere enthalten, ist im Rahmen der erfindungsgemäßen Lehre ganz besonders bevorzugt.

Im Rahmen der erfindungsgemäßen Lehre sind solche Zubereitungen bevorzugt, die zusätzlich mindestens ein Tensid, ausgewählt aus der Gruppe der kationischen, anionischen oder nichtionischen Tenside, enthalten.

Insbesondere wenn die erfindungsgemäßen Zubereitungen als Haarspülungen verwendet werden, ist die Auswahl von Tensiden aus der Gruppe der kationischen Tenside bevorzugt.

Beispiele für die in den erfindungsgemäßen Haarbehandlungsmitteln verwendbaren kationischen Tenside sind insbesondere quartäre Ammoniumverbindungen. Bevorzugt sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil^{R}-Quat 3270 und 3272 (Hersteller: Th. Goldschmidt; diquaternäre Polydimethylsiloxane, Quaternium-80).

Alkylamidoamine, insbesondere Fettsäureamidoamine wie das unter der Bezeichnung Tego Amid^{R}S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quaternäre Esterverbindungen, sogenannte "Esterquats", wie die unter dem Warenzeichen Stepantex^{R} vertriebenen Dialkylammoniummethosulfate und Methyl-hydroxyalkyldialkoyloxyalkyl-ammoniummethosulfate.

Ein Beispiel für ein als kationisches Tensid einsetzbares quaternäres Zuckerderivat stellt das Handelsprodukt Glucquat^{R}100 dar, gemäß CTFA-Nomenklatur ein "Lauryl Methyl Gluceth-10 Hydroxypropyl Dimonium Chloride".

Besonders bevorzugte kationische Tenside sind Alkylamidoamine, quaternäre Esterverbindungen und quaternäre Zuckerderivate.

Erfindungsgemäß verwendete Shampoos enthalten bevorzugt Tenside aus der Gruppe der anionischen Tenside.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ-CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-OSO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül, sowie Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen.

Nichtionogene Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- -: Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- -: C₁₂-C₂₂-Fettsäuremono- und -diester von Anlagerungsprodukten von 1 bis 30 Mol Ethylenoxid an Glycerin,
- -: C₈-C₂₂-Alkylmono- und -oligoglycoside und deren ethoxylierte Analoga sowie
- -: Anlagerungsprodukte von 5 bis 60 Mol Ethylenoxid an Rizinusöl und gehärtetes Rizinusöl.

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Vorzugsweise enthalten die erfindungsgemäß verwendeten Zubereitungen die oberflächenaktiven Verbindungen A in Mengen von 0,5 bis 30 Gew.-%, insbesondere von 1 bis 20 Gew.-%, bezogen auf die jeweilige Zubereitung.

Die erfindungsgemäß verwendeten Zubereitungen weisen bevorzugt einen pH-Wert zwischen 2,5 und 7, insbesondere zwischen 4,0 und 6,0 auf.

Zur Einstellung dieses pH-Wertes kann praktisch jede für kosmetische Zwecke verwendbare Säure verwendet werden. Es kann bevorzugt sein, eine Säure aus der Gruppe der Genußsäuren zu verwenden. Unter Genußsäuren werden solche Säuren verstanden, die im Rahmen der üblichen Nahrungsaufnahme aufgenommen werden und positive Auswirkungen auf den menschlichen Organismus haben. Genußsäuren sind beispielsweise Essigsäure, Milchsäure, Weinsäure, Zitronensäure, Äpfelsäure, Ascorbinsäure und Gluconsäure. Im Rahmen der Erfindung ist die Verwendung von Zitronensäure und Milchsäure besonders bevorzugt.

Bei den erfindungsgemäß verwendeten Haarbehandlungsmitteln handelt es sich bevorzugt um Spülungen, Shampoos, Haarkuren, Färbe- und Tönungsshampoos oder -cremes. Die erfindungsgemäße Wirkstoffkombination kann aber auch in Haarfärbemitteln sowie in Rahmen einer Dauerwellbehandlung eingesetzt werden. Entsprechend dem Verwendungszweck werden die Mittel bevorzugt als wäßrige oder wäßrig-alkoholische Lösungen, Gele, Cremes oder Lotionen formuliert.

Entsprechend dem Verwendungszweck und der Formulierungsart können die erfindungsgemäßen Zubereitungen alle für den jeweiligen Verwendungszweck üblichen kosmetischen Zusätze enthalten.

Solche üblichen Bestandteile sind:
- amphotere Tenside wie beispielsweise N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe,
- zwitterionische Tenside wie beispielsweise die sogenannten Betaine und 2-Alkyl-3-carboxymethyl-3-hydroxyethyl-imidazoline,
- nichtionische Polymere wie beispielsweise Vinylpyrrolidon/Vinylacrylat-Copolymere, Polyvinylpyrrolidon und Vinylpyrrolidon/Vinylacetat-Copolymere,
- direktziehende Farbstoffe
- sogenannte Kuppler- und Entwicklerkomponenten als Oxidationsfarbstoffvorprodukte,
- Reduktionsmittel wie z.B. Thioglykolsäure und deren Derivate, Thiomilchsäure, Cysteamin, Thioäpfelsäure und α-Mercaptoethansulfonsäure,
- Oxidationsmittel wie Wasserstoffperoxid, Kaliumbromat und Natriumbromat,
- Verdickungsmittel wie Agar-Agar, Guar-Gum, Alginate und Xanthan-Gum,
- Strukturanten wie Glucose und Maleinsäure,
- haarkonditionierende Verbindungen wie Phospholipide, beispielsweise Sojalecithin, Ei-Lecitin und Kephaline, sowie Silikonöle,
- Proteinhydrolysate, insbesondere Elastin-, Kollagen-, Keratin-, Milcheiweiß-, Sojaprotein-, Mandelprotein-, Erbsenprotein-, Kartoffelprotein-, Maisprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsvermittler, wie Ethanol, Isopropanol, Ethylenglykol, Propylenglykol, Glycerin und Diethylenglykol,
- Farbstoffe,
- Antischuppenwirkstoffe wie Piroctone Olamine und Zink Omadine,
- weitere Substanzen zur Einstellung des pH-Wertes,
- Wirkstoffe wie Panthenol, Allantoin, Pflanzenextrakte und Vitamine,
- Lichtschutzmittel,
- Konsistenzgeber wie Zuckerester, Polyolester oder Polyolalkylether,
- Fette und Wachse, wie Walrat, Bienenwachs, Montanwachs, Paraffine und Fettalkohole,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex,
- Perlglanzmittel wie Ethylenglykolmono- und -distearat,
- Treibmittel wie Propan-Butan-Gemische, N₂O, Dimethylether, CO₂ und Luft sowie
- Antioxidantien.

Die erfindungsgemäße Wirkstoffkombination wird bevorzugt in solchen Haarbehandlungsmitteln eingesetzt, die nach einer gewissen Einwirkzeit, die in der Regel zwischen einigen Sekunden und ca. 20 Minuten liegt, wieder vom Haar abgespült werden.

Gegenstand der Erfindung ist daher auch ein Verfahren zur Behandlung von Haaren, bei dem das Haar nach Behandlung mit einer Zubereitung gemäß einem der Ansprüche 1 bis 11 anschließend mit Wasser oder einem im wesentlichen Wasser enthaltenden Mittel gespült wird.

Die folgenden Beispiele sollen den Erfindungsgegenstand näher erläutern.

### Beispiele

### I. Farbmetrische Untersuchungen zur Farberhaltung bei colorierten Haaren

### Untersuchungsmethode

Eine Egalisiersträhne (naturweiß, 2 g, ca. 15 cm lang; Fa. Kerling) wurde auf halber Höhe abgebunden. Zur Simulation von stark geschädigtem Haar wurde der untere Teil der Strähne abwechselnd je zweimal kaltgewellt und ultrablondiert. Die Kaltwellung erfolgte jeweils durch Behandlung mit einer wäßrigen Lösung eines Kaltwellmittels auf Basis Ammoniumthioglykolat (30 Minuten) und anschließende Fixierung mit Kaliumbromat-Lösung (10 Minuten). Die Ultrablondierung erfolgt mit einer wäßrigen Lösung von Wasserstoffperoxid und Ammoniumperoxidisulfat. Der obere Teil wurde zur Simulation von wenig geschädigtem Haar lediglich einmal ultrablondiert. Anschließend wurde die gesamte Strähne mit dem Handelsprodukt Poly Diadem Pflege Intensiv Tönung (Nuance Mahagoni-Koralle) (HENKEL) ausgefärbt. Dabei wurden 4 g Färbemischung pro 1 g Haarsträhne eingesetzt. Nach einer Einwirkzeit von 30 Minuten wurden die Strähne mit warmem Wasser (30 °C) gut ausgespült und mit einem Fön getrocknet. Die ausgefärbte Haarsträhne wurde dann 4 Tage bei Raumtemperatur gelagert.

Nach der farbmetrischen Bestimmung der Nullwerte wurde die Haarsträhne 6 mal 1 Minute mit der Testmischung behandelt, wobei die Strähne nach jeder Behandlung gründlich mit Wasser (30 °C) gespült und mit einem Fön getrocknet wurde. Danach erfolgte die farbmetrische Vermessung. Die Messung wurde mit einem Gerät Texflash (Fa. Datacolor) mit Lichtart D65 (Tageslicht) durchgeführt. Dabei wurde die zu vermessende Probe in einer Einspannvorrichtung am Spektralphotometer fixiert und die Remissionswerte über den Bereich des sichtbaren Lichtes von 390 - 700 nm im Abstand von 10 nm gemessen und über einen Rechner verarbeitet. Das Rechnerprogramm ermittelte die Normfarbwerte nach dem CIE-System (Commission Internationale de L'Eclairage) entsprechend DIN 5033 und rechnete sie in Farbabstandszahlen nach DIN 6174 um. Die im weiteren angegebenen Meßwerte sind jeweils Mittelwerte aus 4 Meßpunkten pro Strähnenteil.

Es wurden folgende Größen bestimmt:
- Gesamtfarbabstand DE: (relativ zum Nullwert) des stark geschädigten Teils der Strähne
- Egalisierung E: (Differenz der Gesamtfarbabstände DE nach der Behandlung von stark und wenig geschädigtem Haar)
- Prozentuale Farbstärke S: des stark geschädigten Teils der Strähne (Nullwert = 100 %)

Die Zusammensetzungen der untersuchten Mischungen und die Meßergebnisse sind in Tabelle 1 aufgeführt.

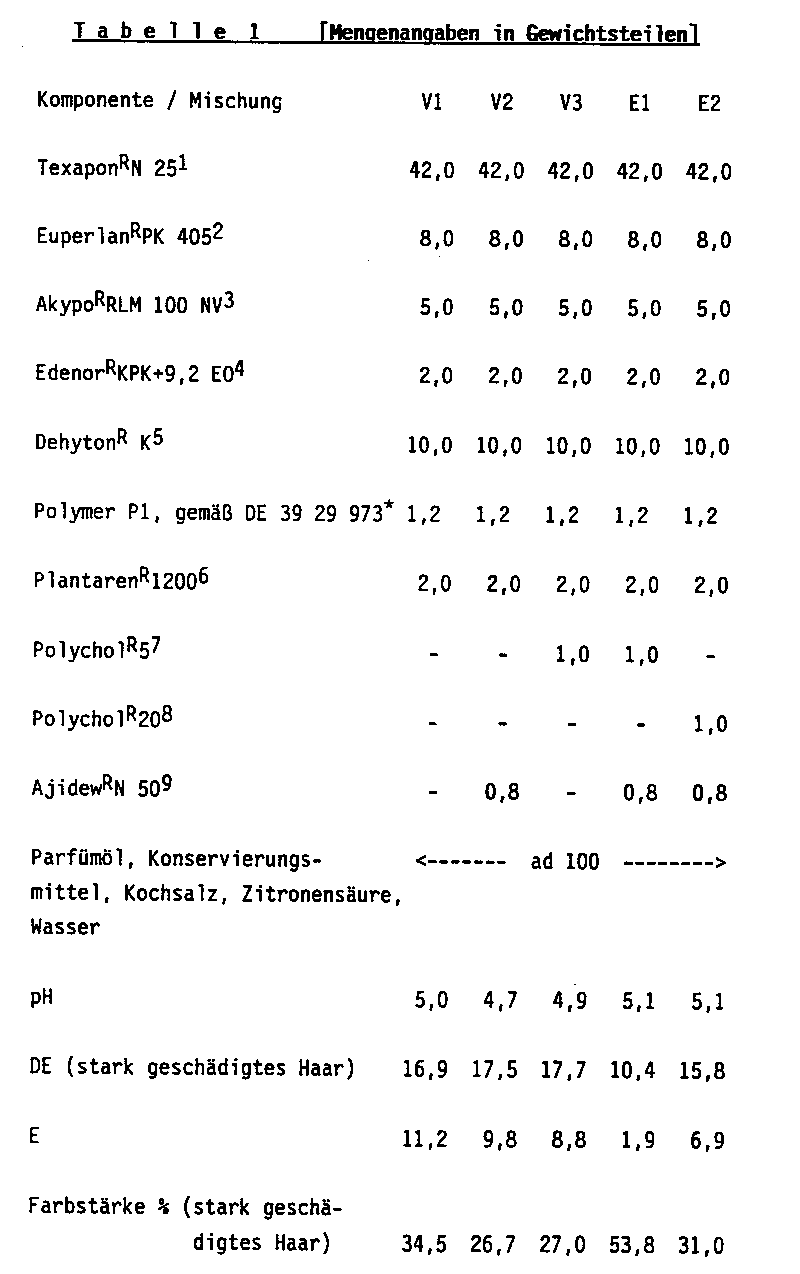

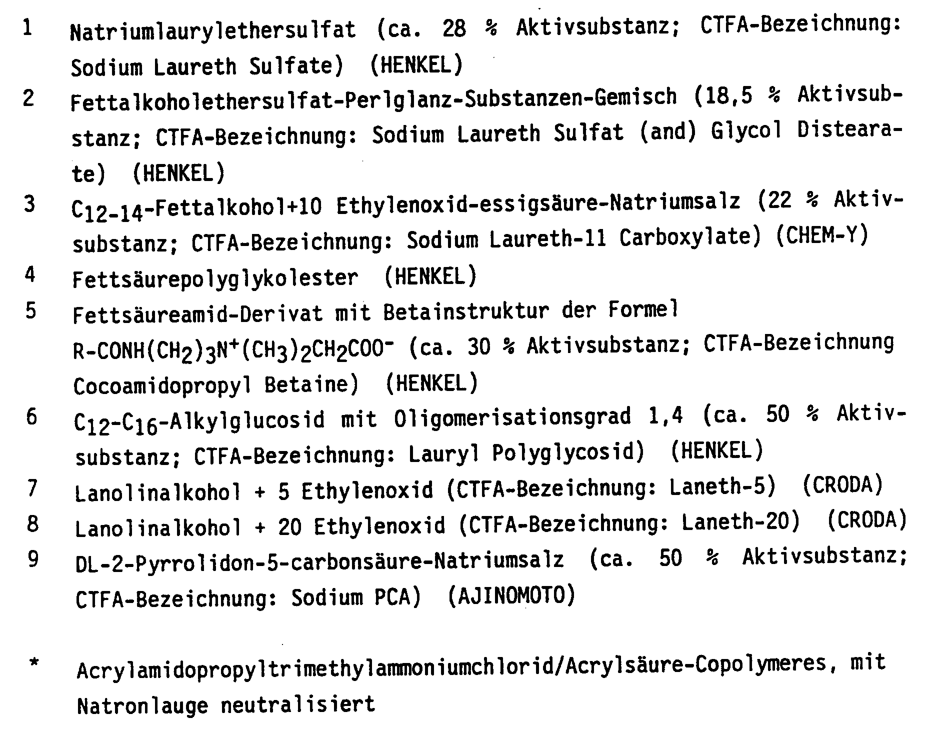

### II. Anwendungsbeispiele

Die Mengenangaben in den folgenden Beispielen sind Gew.-%.

### 1) Haarspülung

| | |
|---|---|
| Stenol^{R}1618¹⁰ | 1,8 |
| Tegoamid^{R}S 18¹¹ | 1,6 |
| 1,2-Propylenglykol | 1,0 |
| Zitronensäure | 0,6 |
| Polychol^{R}5 | 0,5 |
| Ajidew^{R}N 50 | 0,6 |
| Parfümöl, Wasser | ad 100 |

| | |
|---|---|
| ¹⁰ C₁₆/C₁₈-Fettalkohol (HENKEL) | |
| ¹¹ N,N-Dimethyl-N'-stearoyl-1,3-diamino-propan (CTFA-Bezeichnung: Stearamidopropyl Dimethylamin) (GOLDSCHMIDT) | |

### 2) Haarspülung

| | |
|---|---|
| Stenol^{R}1618 | 1,8 |
| Stepantex^{R} VS 90¹² | 1,8 |
| 1,2-Propylenglykol | 0,7 |
| Zitronensäure | 0,9 |
| Polychol^{R}5 | 0,5 |
| Ajidew^{R}N 50 | 0,8 |
| Parfümöl, Farbstoff, Wasser | ad 100 |

| | |
|---|---|
| ¹² N-Methyl-N(2-hydroxyethyl)-N,N-di(talgacyloxyethyl)ammoniummethosulfat (90% Aktivsubstanz in Isopropanol) (STEPAN) | |

### 3) Haarspülung

| | |
|---|---|
| Stenol^{R}1618 | 2,5 |
| Eumulgin^{R}B 1¹³ | 1,0 |
| Eumulgin^{R}B 2¹⁴ | 1,0 |
| Cutina^{R}CP¹⁵ | 1,0 |
| Eutanol^{R}G¹⁶ | 0,5 |
| Polawax^{R}GP 200¹⁷ | 0,75 |
| Abil^{R}Quat 3270¹⁸ | 0,5 |
| Dow Corning^{R}929-Emulsion¹⁹ | 2,6 |
| Polychol^{R}5 | 1,0 |
| Ajidew^{R}N 50 | 1,5 |
| Zitronensäure | 0,02 |
| Wasser | ad 100 |

| | |
|---|---|
| ¹³ Cetylstearylalkohol mit ca. 12 Mol EO (CTFA-Bezeichnung: Ceteareth-12) (HENKEL) | |
| ¹⁴ Cetylstearylalkohol mit ca. 20 Mol EO (CTFA-Bezeichnung: Ceteareth-20) (HENKEL) | |
| ¹⁵ Ester aus gesättigten, langkettigen Fettalkoholen und Fettsäuren, vornehmlich Palmitinsäurecetylester (CTFA-Bezeichnung: Cetyl Palmitate) (HENKEL) | |
| ¹⁶ Kondensationsprodukt aus gesättigten flüssigen Fettalkoholen, vorwiegen Decylalkohol, hergestellt nach der Guerbet-Reaktion (CTFA-Bezeichnung: Octyldodecanol) (HENKEL) | |
| ¹⁷ Stearylalkohol-Polyethylenglykolstearat-Gemisch (CTFA-Bezeichnung: Stearyl Alkohol (and) PEG-Stearate) (CRODA) | |
| ¹⁸ Diquaternäres Polymethylsiloxane (CTFA-Bezeichnung: Quaternium-80) (GOLDSCHMIDT) | |
| ¹⁹ amino-funktionelles Polydimethylsiloxan (35 % Aktivsubstanz) (DOW CORNING) | |

### 4) Haarspülung

| | |
|---|---|
| Rewoquat^{R}W 7500²⁰ | 1,1 |
| Lanette O²¹ | 3,0 |
| Eumulgin^{R}B 1 | 0,8 |
| Eumulgin^{R}B 2 | 1,6 |
| Cutina^{R}GMS²² | 0,5 |
| Eutanol^{R}G | 1,0 |
| Polychol^{R}5 | 2,0 |
| Ajidew^{R}N 50 | 1,5 |
| Wasser | ad 100 |

| | |
|---|---|
| ²⁰ 1-Methyl-2-nortalgalkyl-3-talgfettsäure-amidoethyl-imidazolinium-methosulfat (ca. 75 % Aktivsubstanz) (REWO) | |
| ²¹ Gemisch höherer, gesättigter Fettalkohole, vorwiegend Cetyl- und Stearylalkohol (CTFA-Bezeichnung: Cetearyl Alcohol) (HENKEL) | |
| ²² Glycerinmonostearat (CTFA-Bezeichnung: Glyceryl Stearate) (HENKEL) | |

### 5) Shampoo

| | |
|---|---|
| Texapon^{R}N 25 | 43,0 |
| Dehyton^{R}K | 10,0 |
| Plantaren^{R}-1200 | 4,0 |
| Euperlan^{R}PK 3000 ²³ | 1,6 |
| Arquad^{R}316²⁴ | 0,8 |
| Polychol^{R}20 | 3,0 |
| Ajidew^{R}N 50 | 4,0 |
| Glucamate^{R}DOE 120²⁵ | 0,5 |
| Natriumchlorid | 0,2 |
| Wasser | ad 100 |

| | |
|---|---|
| ²³ Flüssige Dispersion von perlglanzgebenden Substanzen und Amphotensid (ca. 62 % Aktivsubstanz; CTFA-Bezeichnung: Glycol Distearate (and) Glycerin (and) Laureth-4 (and) Cocoamidopropyl Betaine) (HENKEL) | |
| ²⁴ Tri-C₁₆-alkylmethylammoniumchlorid (AKZO) | |
| ²⁵ ethoxyliertes Methylglucosid-dioleat (CTFA-Bezeichnung: PEG-120 Methyl Glucose Dioleate) (AMERCHOL) | |

### 6) Shampoo

| | |
|---|---|
| Texapon^{R}N 70²⁶ | 21,0 |
| Plantaren^{R}-1200 | 8,0 |
| Genamin^{R}DSAC²⁷ | 1,2 |
| Cutina^{R}EGMS²⁸ | 0,6 |
| Polychol^{R}20 | 2,0 |
| Ajidew A 100²⁹ | 2,8 |
| Antil ^{R}141³⁰ | 1,3 |
| Natriumchlorid | 0,2 |
| Magnesiumhydroxid | ad pH 4,5 |
| Wasser | ad 100 |

| | |
|---|---|
| ²⁶ Natriumlaurylethersulfat (ca. 72 % Aktivsubstanz) (HENKEL) | |
| ²⁷ Dimethyldistearylammoniumchlorid (HOECHST) | |
| ²⁸ Ethylenglykolmonostearat (ca. 25-35% Monoester, 60-70% Diester; CTFA-Bezeichnung: Glycol Stearate) (HENKEL) | |
| ²⁹ DL-2-Pyrrolidon-5-carbonsäure (CTFA-Bezeichnung: PCA) (AJINOMOTO) | |
| ³⁰ Polyoxyethylen-propylenglykoldioleat (40 % Aktivsubstanz; CTFA-Bezeichnung: Propylene Glycol (and) PEG-55 Propylene Glycol Oleate) (GOLDSCHMIDT) | |

### 7) Shampoo

| | |
|---|---|
| Texapon^{R}K 14 S³¹ | 50,0 |
| Dehyton^{R}K | 10,0 |
| Akypo^{R}RLM 100 NV | 4,5 |
| Polymer P1, entsprechend DE 39 29 973 | 0,6 |
| Cutina^{R}AGS³² | 2,0 |
| D-Panthenol | 0,5 |
| Glucose | 1,0 |
| Salicylsäure | 0,4 |
| Natriumchlorid | 0,5 |
| Polychol^{R}20 | 2,5 |
| Ajidew^{R}N 50 | 2,0 |
| Wasser | ad 100 |

| | |
|---|---|
| ³¹ Natriumlaurylmyristylethersulfat (ca. 28 % Aktivsubstanz; CTFA-Bezeichnung: Sodium Myreth Sulfate) (HENKEL) | |
| ³² Ethylenglykolstearat (ca. 5-15% Monoester, 85-95% Diester; CTFA-Bezeichnung: Glycol Distearate) (HENKEL) | |

### 8) Shampoo

| | |
|---|---|
| Texapon^{R}K 14 S | 25,0 |
| Texapon^{R}SB 3³³ | 7,5 |
| Eucarol^{R}TA³⁴ | 12,0 |
| Akypo^{R}RLM 100 NV | 9,0 |
| Dehyton^{R}AB 30³⁵ | 8,3 |
| Polymer P1, entsprechend DE 39 29 973 | 0,6 |
| Elfacos^{R}GT 282S³⁶ | 0,5 |
| Polychol^{R}20 | 4,0 |
| Ajidew A 100 | 2,8 |
| Natriumchlorid | 0,5 |
| KOH | ad pH 5,0 |
| Wasser | ad 100 |

| | |
|---|---|
| ³³ Sulfobernsteinsäurehalbester auf Basis eines Alkylpolyglycolethers, Di-Na-Salz (ca. 40 % Aktivsubstanz; CTFA-Bezeichnung: Disodium Laurethsulfosuccinat) (HENKEL) | |
| ³⁴ Laurylalkohol+7 Ethylenoxid-tartrat-Natriumsalz (ca. 25 % Aktivsubstanz; CTFA-Bezeichnung: Sodium Laureth-7 Tartrate) (AUSICHEM) | |
| ³⁵ Fettamin-Derivat mit Betainstruktur (ca. 30 % Aktivsubstanz; CTFA-Bezeichnung: Coco-Betaine) (HENKEL) | |
| ³⁶ Talgalkohol+60 Ethylenoxid-myristylether (CTFA-Bezeichnung: Talloweth-60 Myristyl Glycol) (AKZO) | |

### 9) Kurpackung (abspülbar)

| | |
|---|---|
| Stenol^{R}1618 | 3,0 |
| Eumulgin^{R}B 1 | 0,5 |
| Eumulgin^{R}B 2 | 0,5 |
| Cutina^{R}CP | 1,0 |
| Eutanol^{R}G | 1,5 |
| Hostacerin^{R}PN 73³⁷ | 0,004 |
| Dow Corning^{R}929-Emulsion | 2,9 |
| Polychol^{R}5 | 1,5 |
| Ajidew^{R}N 50 | 2,0 |
| Wasser | ad 100 |

| | |
|---|---|
| ³⁷ Polyacrylsäure-Natriumsalz-Copolymer (HOECHST) | |

### 10) Kurpackung (auf dem Haar verbleibend)

| | |
|---|---|
| Celquat^{R}L 200³⁸ | 0,6 |
| Luviskol^{R}K30³⁹ | 0,2 |
| D-Panthenol | 0,5 |
| Polymer P1, entsprechend DE 39 29 973 | 0,6 |
| Dehyquart^{R}SP⁴⁰ | 1,0 |
| Nutrilan^{R}I⁴¹ | 1,0 |
| Natrosol^{R}250 HR⁴² | 1,1 |
| Polychol^{R}5 | 1,5 |
| Ajidew^{R}N 50 | 2,0 |
| Wasser | ad 100 |

| | |
|---|---|
| ³⁸ quaterniertes Cellulose-Derivat (95 % Aktivsubstanz; CTFA-Bezeichnung: Polyquaternium-4) (DELFT NATIONAL) | |
| ³⁹ Polyvinylpyrrolidon (95 % Aktivsubstanz; CTFA-Bezeichnung: PVP) (BASF) | |
| ⁴⁰ Wäßrige Lösung von Oxyethylalkylammoniumphosphat (ca. 50 % Aktivsubstanz; CTFA-Bezeichnung: Quaternium-52) (HENKEL) | |
| ⁴¹ Kollagenhydrolysat (ca. 39 % Aktivsubstanz; CTFA-Bezeichnung: Hydrolyzed Collagen) (HENKEL) | |
| ⁴² Hydroxyethylcellulose (AQUALON) | |

### 11) Farbecreme

| | |
|---|---|
| C₁₂₋₁₈-Fettalkohol | 1,2 |
| Lanette O | 4,0 |
| Eumulgin^{R}B 2 | 0,8 |
| Cutina^{R}KD 16⁴³ | 2,0 |
| Natriumsulfit | 0,5 |
| L(+)-Ascorbinsäure | 0,5 |
| Ammoniumsulfat | 0,5 |
| 1,2-Propylenglykol | 1,2 |
| Polymer JR^{R}400⁴⁴ | 0,3 |
| p-Aminophenol | 0,35 |
| p-Toluylendiamin | 0,85 |
| 2-Methylresorcin | 0,14 |
| 6-Methyl-m-aminophenol | 0,42 |
| Polychol^{R}5 | 0,5 |
| Ajidew^{R}N 50 | 1,2 |
| Ammoniak | 1,5 |
| Wasser | ad 100 |

| | |
|---|---|
| ⁴³ Fettsäuremono/diglycerid-Emulgator-Gemisch (CTFA-Bezeichnung: Tallow Glycerides (and) Glyceryl Stearate (and) Potassium Stearate) (HENKEL) | |
| ⁴⁴ quaternierte Hydroxyethylcellulose (Union Carbide) | |

### 12) Entwicklerdispersion für Färbecreme 11)

| | |
|---|---|
| Texapon^{R}N 25 | 2,1 |
| Wasserstoffperoxid (50%ig) | 12,0 |
| Turpinal^{R}SL⁴⁵ | 1,7 |
| Latekoll^{R}D⁴⁶ | 12,0 |
| Polychol^{R}20 | 0,9 |
| Ajidew^{R}N 50 | 0,3 |
| Wasser | ad 100 |

| | |
|---|---|
| ⁴⁵ 1-Hydroxyethan-1,1-diphosphonsäure (60 % Aktivsubstanz; CTFA-Bezeichnung: Etidronic Acid) (HENKEL) | |
| ⁴⁶ Acrylester-Methacrylsäure-Copolymer (25 % Aktivsubstanz) (BASF) | |

Die Färbecreme hatte einen pH-Wert von 10,0. Sie bewirkte eine intensive rote Tönung des Haares.

### 13) Tönungsshampoo

| | |
|---|---|
| Texapon^{R}N 70 | 14,0 |
| Dehyton^{R}K | 10,0 |
| Akypo^{R}RLM 45 NV⁴⁷ | 14,7 |
| Plantaren^{R}-1200 | 4,0 |
| Polymer P1, entsprechend DE 39 29 973 | 0,3 |
| Cremophor^{R}RH 40⁴⁸ | 0,8 |
| Farbstoff C.I. 12 719 | 0,02 |
| Farbstoff C.I. 12 251 | 0,02 |
| Farbstoff C.I. 12 250 | 0,04 |
| Farbstoff C.I. 56 059 | 0,03 |
| PHB-Ester | 0,25 |
| Parfümöl | q.s. |
| Polychol^{R}20 | 1,6 |
| Ajidew^{R}N 50 | 1,2 |
| Wasser | ad 100 |

| | |
|---|---|
| ⁴⁷ Laurylalkohol+4,5 Ethylenoxid-essigsäure-Natriumsalz (20,4 % Aktivsubstanz) (CHEM-Y) | |
| ⁴⁸ Rizinus-Öl, hydriert + 45 Ethylenoxid (CTFA-Bezeichnung: PEG-40 Hydrogenated Castor Oil) (BASF) | |

Beim Waschen der Haare mit diesem Tönungs-Shampoo erhalten diese einen glänzenden, hellblonden Farbton.

### 17) Cremedauerwelle

| Wellcreme | |
|---|---|
| Plantaren^{R}-800⁴⁹ | 5,0 |
| Thioglykolsäure | 8,0 |
| Turpinal^{R}SL | 0,5 |
| Ammoniak (25%ig) | 7,3 |
| Ammoniumcarbonat | 3,0 |
| Cetyl/Stearyl-Alkohol | 5,0 |
| Guerbet-Alkohol | 4,0 |
| Polychol^{R}5 | 3,0 |
| Ajidew^{R}N 50 | 2,0 |
| Parfümöl | q.s. |
| Wasser | ad 100 |

| | |
|---|---|
| ⁴⁹ C₈-C₁₀-Alkylglucosid mit Oligomerisationsgrad 1,6 (ca. 60% Aktivsubstanz) (HENKEL) | |

| Fixierlösung | |
|---|---|
| Plantaren^{R}-800 | 5,0 |
| gehärtetes Rizinusöl | 2,0 |
| Kaliumbromat | 3,5 |
| Nitrilotriessigsäure | 0,3 |
| Zitronensäure | 0,2 |
| Merquat^{R}550⁵⁰ | 0,5 |
| Polychol^{R}20 | 1,5 |
| Ajidew^{R}A 100 | 0,5 |
| Parfümöl | q.s. |
| Wasser | ad 100 |

| | |
|---|---|
| ⁵⁰ Dimethyldiallylammoniumchlorid-Acrylamid-Copolymer (8 % Aktivsubstanz; CTFA-Bezeichnung: Polyquarternium 7) (MOBIL OIL) | |

## Patentansprüche

1. Verwendung einer wäßrigen Zubereitung enthaltend eine Wirkstoffkombination, bestehend aus
(A) alkoxylierten Wollwachsalkoholen und
(B) einer Pyrrolidoncarbonsäure und/oder einem Pyrrolidoncarbonsäurederivat der allgemeinen Formel (I), 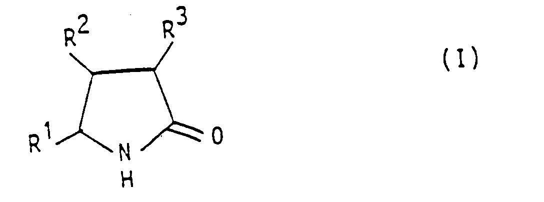 in der mindestens einer der Substituenten R¹ bis R³ steht für eine Gruppe -COOR⁴, in der R⁴ Wasserstoff, ein Alkalimetallion, ein Erdalkalimetallion oder ein Ammoniumion ⁺NHR⁵R⁶R⁷ ist, in dem R⁵ bis R⁷ unabhängig voneinander Wasserstoff, Alkylgruppen mit 1 bis 22 C-Atomen, Hydroxyalkylgruppen mit 1 bis 4 C-Atomen, Alkenylgruppen mit 2 bis 22 C-Atomen, Acylgruppen mit 2 bis 22 C-Atomen oder gegebenenfalls substituierte aromatische Gruppen mit 6 bis 10 C-Atomen sind, und die restlichen Substituenten R¹ bis R³ für Wasserstoff oder Alkylgruppen mit 1 bis 4 C-Atomen stehen,
sowie üblichen kosmetischen Bestandteilen zur Behandlung, insbesondere zur Reinigung und Pflege, von Haaren.

2. Verwendung nach Anspruch 1, dadurch gekennzeichnet, daß der alkoxylierte Wollwachsalkohol (A) 1 bis 50 Moleküle Ethylenoxid pro Alkoholmolekül enthält.

3. Verwendung nach einem der Ansprüche 1 oder 2, dadurch gekennzeichnet, daß die Verbindung (B) der allgemeinen Formel (I) 2-Pyrrolidon-5-carbonsäure oder ein Salz dieser Säure ist.

4. Verwendung nach Anspruch 3, dadurch gekennzeichnet, daß die Verbindung (B) der allgemeinen Formel (I) das Natriumsalz der 2-Pyrrolidon-5-carbonsäure ist.

5. Verwendung nach einem der Ansprüche 1 bis 4, dadurch gekennzeichnet, daß die Zubereitung 0,1 - 5 Gew.-% alkoxylierte Wollwachsalkohole (A) und 0,1 - 5 Gew.-% der Verbindung (B) enthält.

6. Verwendung nach einem der Ansprüche 1 bis 5, dadurch gekennzeichnet, daß die Zubereitung zusätzlich ein anionisches, kationisches oder amphoteres Polymer enthält.

7. Verwendung nach Anspruch 6, dadurch gekennzeichnet, daß die Zubereitung ein amphoteres Polymer enthält.

8. Verwendung nach einem der Ansprüche 1 bis 7, dadurch gekennzeichnet, daß die Zubereitung zusätzlich ein Tensid, ausgewählt aus der Gruppe der kationischen, anionischen oder nichtionischen Tenside, enthält.

9. Verwendung nach Anspruch 8, dadurch gekennzeichnet, daß die Zubereitung ein kationisches Tensid, ausgewählt aus der Gruppe der Amidoamine, quaternären Esterverbindungen und quaternären Zuckerderivate, enthält.

10. Verwendung nach Anspruch 8, dadurch gekennzeichnet, daß die Zubereitung ein anionisches Tensid, ausgewählt aus der Gruppe der Alkylsulfat, Alkylethersulfate, Ethercarbonsäuren und Sulfobernsteinsäure- mono/und -dialkylester enthält.

11. Verwendung nach einem der Ansprüche 1 bis 10, dadurch gekennzeichnet, daß die Zubereitung einen pH-Wert zwischen 2,5 und 7 aufweist.

12. Verfahren zur Behandlung von Haaren, dadurch gekennzeichnet, daß das Haar nach Behandlung mit einer Zubereitung gemäß einem der Ansprüche 1 bis 11 anschließend mit Wasser oder einem im wesentlichen Wasser enthaltenden Mittel gespült wird.

## Claims

1. The use of a water-based preparation containing a combination of active ingredients consisting of
(A) alkoxylated wool wax alcohols and
(B) a pyrrolidone carboxylic acid and/or pyrrolidone carboxylic acid derivative corresponding to general formula (I) 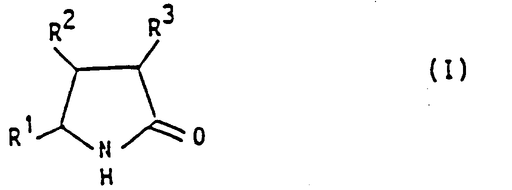 in which at least one of the substituents R¹ to R³ is a group -COOR⁴, where R⁴ is hydrogen, an alkali metal ion, an alkaline earth metal ion or an ammonium ion ⁺NHR⁵R⁶R⁷, where R⁵ to R⁷ independently of one another represent hydrogen, C₁₋₂₂ alkyl groups, C₁₋₄ hydroxyalkyl groups, C₂₋₂₂ alkenyl groups, C₂₋₂₂ acyl groups or optionally substituted C₆₋₁₀ aromatic groups, and the remaining substituents R¹ to R³ are hydrogen or C₁₋₄ alkyl groups,
and typical cosmetic constituents for the treatment of hair, more particularly for the washing and care of hair.

2. The use claimed in claim 1, characterized in that the alkoxylated wool wax alcohol (A) contains 1 to 50 molecules ethylene oxide per alcohol molecule.

3. The use claimed in claim 1 or 2, characterized in that compound (B) corresponding to general formula (I) is 2-pyrrolidone-5-carboxylic acid or a salt of this acid.

4. The use claimed in claim 3, characterized in that compound (B) corresponding to general formula (I) is the sodium salt of 2-pyrrolidone-5-carboxylic acid.

5. The use claimed in any of claims 1 to 4, characterized in that the preparation contains 0.1 to 5% by weight alkoxylated wool wax alcohols (A) and 0.1 to 5% by weight of compound (B).

6. The use claimed in any of claims 1 to 5, characterized in that the preparation additionally contains an anionic, cationic or amphoteric polymer.

7. The use claimed in claim 6, characterized in that the preparation contains an amphoteric polymer.

8. The use claimed in any of claims 1 to 7, characterized in that the preparation additionally contains a surfactant selected from the group consisting of cationic, anionic or nonionic surfactants.

9. The use claimed in claim 8, characterized in that the preparation contains a cationic surfactant selected from the group consisting of amidoamines, quaternary ester compounds and quaternary sugar derivatives.

10. The use claimed in claim 8, characterized in that the preparation contains an anionic surfactant selected from the group consisting of alkyl sulfate, alkyl ether sulfates, ether carboxylic acids and sulfosuccinic acid mono- and dialkyl esters.

11. The use claimed in any of claims 1 to 10, characterized in that the preparation has a pH value of 2.5 to 7.

12. A process for treating hair, characterized in that, after treatment with the preparation claimed in any of claims 1 to 11, the hair is rinsed with water or with a preparation largely containing water.

## Revendications

1. Utilisation d'une préparation aqueuse renfermant une association de principes actifs, constituée:
(A) d'alcools de graisse de laine alcoxylé et
(B) d'un acide pyrrolidonecarboxylique et/ou d'un
dérivé de l'acide pyrrolidonecarboxylique de la formule générale (I) 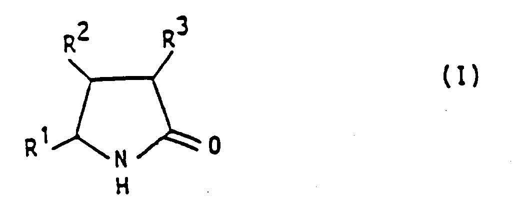 dans laquelle, au moins un des substituants R¹ à R³ représente un groupe -COOR⁴, dans lequel R⁴ est l'hydrogène, un ion de métal alcalin, un on de métal alcalino-terreux ou un ion d'ammonium ⁺NHR⁵R⁶R⁷, dans lequel R⁵ à R⁷ représentent indépendamment l'un de l'autre l'hydrogène, des groupes alkyle comportant 1 à 22 atomes de C, des groupes hydroxyalkyle possédant 1 à 4 atomes de C, des groupes alcényle comprenant 2 à 22 atomes de C, des groupes acyle avec 2 à 22 atomes de C ou, le cas échéant, des groupes aromatiques substitués comportant 6 à 10 atomes de C, et les substituants restants R¹ à R³ correspondent à l'hydrogène ou à des groupes alkyle comportant 1 à 4 atomes de C,
ainsi que de constituants cosmétiques usuels, pour le traitement, en particulier pour le lavage et le soin des cheveux.

2. Utilisation selon la revendication 1, caractérisée en ce que l'alcool de graisse de laine alcoxylé (A) renferme 1 à 50 molécules d'oxyde d'éthyléne par molécule d'alcool.

3. Utilisation selon une des revendications 1 ou 2, caractérisée en ce que le composé (B) de la formule générale (I) est un acide 2-pyrrolidone-5-carboxylique ou un sel de cet acide.

4. Utilisation selon la revendication 3, caractérisée en ce que le composé (B) de la formule générale (I) est le sel sodique de l'acide 2-pyrrolidone-5-carboxylique.

5. Utilisation selon une des revendications 1 à 4, caractérisée en ce que la préparation contient 0,1 à 5 % en poids d'alcools de graisse de laine alcoxylés (A) et 0,1 à 5 % en poids du composé (B).

6. Utilisation selon une des revendications 1 à 5, caractérisée en ce que la préparation contient en plus un polymère anionique, cationique ou amphotère.

7. Utilisation selon la revendication 6, caractérisée en ce que la préparation contient un polymère amphothère.

8. Utilisation selon une des revendications 1 à 7 caractérisée en ce que la préparation contient en plus un tensioactif, sélectionné parmi le groupe formé des surfactifs cationiques, anioniques ou non ioniques.

9. Utilisation selon la revendication 8, caractérisée en ce que la préparation contient un tensioactif cationique, sélectionné parmi le groupe constitué des amidoamines, des composés d'esters quaternaires et des dérivés de sucre quaternaires.

10. Utilisation selon la revendication 8, caractérisée en ce que la préparation contient un tensioactif anionique, sélectionné parmi le groupe constitué des alkylsulfates, des alkyléthersulfates, des acides éthercarboxyliques et des mono- et des dialkylesters de l'acide sulfosuccinique.

11. Utilisation selon une des revendications 1 à 10, caractérisée en ce que la préparation présente un pH compris entre 2,5 et 7.

12. Procédé de traitement des cheveux, caractérisé en ce que les cheveux sont rincés à l'eau ou avec un produit contenant essentiellement de l'eau après traitement par une préparation conforme à une des revendications 1 à 11.
